# EUROPEAN PATENT SPECIFICATION

(11) **EP 4 214 072 B1**
(45) Date of publication and mention of the grant of the patent: **15.04.2026**
(21) Application number: 21770351.1
(22) Date of filing: 20.08.2021
(51) Int. Cl.: B60H 1/00, B60H 3/00, A61L 9/20

(54) **VEHICLE OCCUPANT PROTECTION**
SCHUTZ FÜR FAHRZEUGINSASSEN
PROTECTION D'OCCUPANT DE VÉHICULE

(30) Priority: 21.09.2020 US 202017026547
(43) Date of publication of application: 26.07.2023
(73) Proprietor: International Truck Intellectual Property Company, LLC, Lisle, IL 60532 (US)
(72) Inventor: SORENSEN, Tom Lee, Naperville, Illinois 60564 (US)
(74) Representative: Kuhnen & Wacker Patent- und Rechtsanwaltsbüro PartG mbB
(86) International application number: PCT/US2021/046845
(87) International publication number: WO 2022/060526

(56) References cited:
- WO-A1-2006/016346
- US-A1- 2019 381 863
- US-A1- 2020 061 223
- US-A1- 2020 254 132

## Description

### BACKGROUND

This invention relates generally to a system and a method of protecting an occupant of a vehicle. More specifically, this invention relates to a system and a method protecting an occupant of a school bus, a commercial vehicle and the like, from an airborne contaminant.

Currently, many vehicles are ventilated mechanically with an HVAC system that circulates air within a cabin of the vehicle. This circulated air is shared among occupants, such as a driver and passengers of a school bus, multiple drivers of a commercial vehicle, and the like of the vehicle. Any of these occupants can introduce a contaminant to circulated air thereby contaminating the HVAC system, a vehicle passenger cabin, and a vehicle driver cabin. Furthermore, elements of a vehicle, such as an air conditioner coil and the like, may present an attractive environment for a biological contaminant, such as a microorganism, viz. bacteria, viruses, fungi and parasites. Some contaminants may be incorporated into the circulated air. Anything incorporated into the circulated air can impact an occupant of the vehicle. Additionally, a contaminant may be present in air outside of the vehicle. This contaminant can be introduced into the vehicle, e.g. when a door or a window on the vehicle is opened. Accordingly, it is desirable to have a system and a method that protects an occupant of a vehicle from an airborne contaminant.

US 2019381863 A1 discloses a system in accordance with the preamble of claim 1. In particular a method for ventilating a vehicle interior compartment is taught, that includes at least intermittently, setting an air quality mode in which fresh air and/or circulated air is supplied to the vehicle interior compartment and controlling an interior compartment pressure in the vehicle interior compartment through variation of the flow resistance of an interior compartment ventilation device in an open-loop and/or a closed-loop manner such that the interior compartment pressure is higher than an ambient pressure outside the vehicle interior compartment.

### SUMMARY

The present invention is defined in claim 1. Further improvements are subject to the dependent claims.

### DESCRIPTION OF THE DRAWINGS

Fig. I is a generic schematic diagram of a vehicle HVAC system including an embodiment of a system for protecting an occupant of a vehicle disclosed herein; and

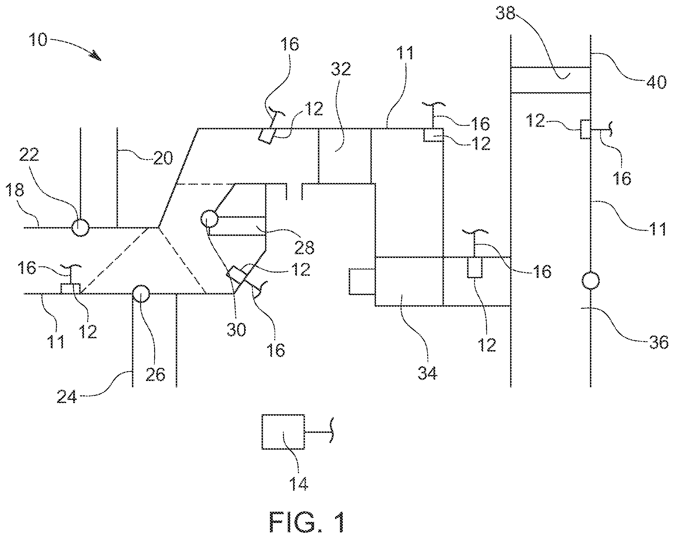

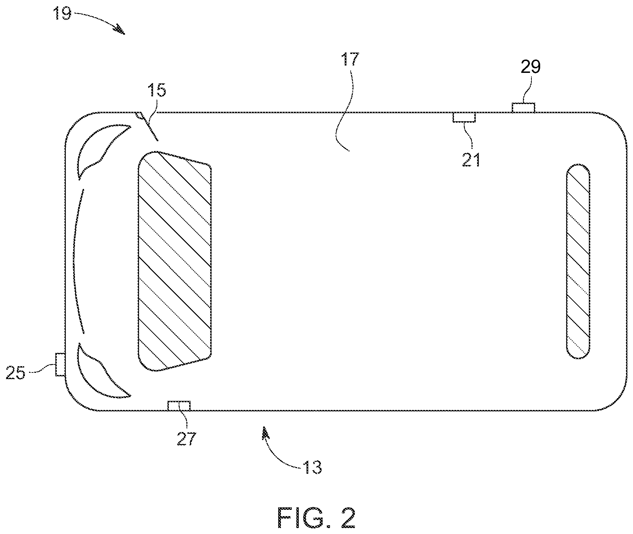

Fig. 2 is a generic view of a vehicle including the system of Fig. 1.

### DETAILED DESCRIPTION

This invention relates specifically to a system and a method for protecting an occupant of a vehicle, such as a school bus, a commercial vehicle and the like. Embodiments disclosed herein generally comprise an HVAC system 10 disposed on a vehicle 13 comprising a duct 11 disposed in the vehicle 13. The system for sterilizing or reducing contaminant in air in the vehicle 13 includes at least one source 12 of electromagnetic radiation located in the duct 11 of the HVAC system 10. Other elements of the HVAC system 10 include, but are not limited to, duct 18 that conveys air to a vent in the vehicle 13, a duct 20 that conveys air to a vent associated with a defroster, an operable door 22 that controls air movement to the duct 20, a duct 24 that conveys air to a vent on the vehiclel3, an operable door 26 that controls air movement to the duct 24, a heater core 28 that imparts thermal energy to air, an operable door 30 that controls air movement from the heater core 28, an evaporator core 32 that absorbs thermal energy from air, a blower 34 that moves air, an operable door 36 that controls air movement into a cabin of the vehicle 13, an air filter 38, a duct 40 that conveys air from an exterior 19 of the vehicle 13, and a duct 36 that conveys air to a cabin of the vehicle 13.

As shown in Fig. 2, the vehicle 13 includes at least one portal 15, such as a door, a window and the like, through which air passes between an interior 17 of the vehicle 13 and an exterior 19 of the vehicle 13. At least one sensor 21 is disposed on the interior 15 of the vehicle 13, and at least one sensor 25 is disposed on the exterior 19 of the vehicle 13. Both of the at least one sensors 21 and 25 sample air adjacent the at least one sensors 21 and 25 to monitor quality of that air including, but not limited to, presence of an airborne contaminant, such as carbon monoxide, carbon dioxide, diesel particulate, pollen, viruses, bacteria, vapors, aerosols and the like, adjacent the at least one sensors 21 and 25. The vehicle 13 includes at least one pressure sensor 27 disposed on the interior 17 of the vehicle 13 and at least one pressure sensor 29 disposed on the exterior 19 of the vehicle 13. The at least one pressure sensors 27 and 29 monitor pressure of air adjacent the at least one pressure sensors 27 and 29, respectively. The at least one sensors 21 and 25 and the at least one pressure sensors 27 and 29 are connected, wired, wirelessly or otherwise, to a controller 14, which can comprise one or more data processors, on the vehicle 13. In some embodiments, the controller 14 is augmented by at least one data processor located outside of the vehicle 13 and connected with the vehicle 13 by appropriate means, such as wireless connection to the internet and the like. In some embodiments, the at least one sensors 21 and 27 disposed on the interior 17 of the vehicle 13 may comprise a single sensor. Also, in some embodiments, the at least one sensors 25 and 29 disposed on the exterior 19 of the vehicle 13 may comprise a single sensor.

Returning to Fig. 1, in some embodiments, the at least one source 12 comprises at least one of a light emitting diode, low power light emitting diode, a mercury vapor light and the like. There are six (6) at least one sources 12 in the embodiment shown in Fig. 1. The electromagnetic radiation has a wavelength within the range of about 200 nanometers to about 280 nanometers, with 260 nanometers being an absorbance wavelength of some nucleic acids. The at least one sources 12 are connected with the controller 14 by a conductor 16, either wired or wirelessly. The controller 14 is disposed on the vehicle. In some embodiments, the controller 14 comprises a body controller of a vehicle. The at least one sources 12 are positioned within the HVAC system 10 at locations empirically determined to reduce contaminant in air circulating within the HVAC system 10 and to sterilize portions of the HVAC system 10 illuminated by the electromagnetic radiation from the at least one sources 12. Locations of the at least one sources 12 are chosen to reduce likelihood of unintended exposure to the electromagnetic radiation from the at least one sources 12, i.e. reduce likelihood of exposure of the vehicle occupant to the electromagnetic radiation.

The controller 14 determines operation of the at least one sources 12, viz. time when the at least one sources 12 emit electromagnetic radiation. The controller 14 can determine intensity of the electromagnetic radiation. The controller 14 can be programmable. The controller 14 can allow the at least one sources 12 to emit electromagnetic radiation, for instance, at specific times of day, for specific temporal duration, etc. The controller 14 may allow the at least one sources 12 to emit electromagnetic radiation at specific times to manage vehicle power usage. In some embodiments, the controller 14 may allow the at least one sources 12 to emit electromagnetic radiation substantially continuously thereby enabling substantially continuous sterilization or contaminant reduction of the HVAC system 10.

With structure of a system for reducing contaminant in air in a vehicle 13 being described, now attention is drawn to a method of reducing contaminant in air in a vehicle 13.

Once the vehicle 13 includes an HVAC system 10 with at least one source 12 of electromagnetic radiation having a wavelength within a range of about 200 nanometers to about 280 nanometers located in a duct 11 of the HVAC system 10, then temporal duration of emission of the electromagnetic radiation from the at least one source 12 of electromagnetic radiation and intensity of electromagnetic radiation are determined. These parameters are inputted into the controller 14. The controller 14 energizes the at least one source 12 of electromagnetic radiation in accordance with the inputted parameters.

The at least one sensor 25 samples air outside of the vehicle 13 periodically for air quality and reports results to the controller 14. Similarly, the at least one sensor 21 on the interior 17 of the vehicle 13 periodically samples quality of the air on the interior 17 of the vehicle 13 and reports results to the controller 14. The controller 14 compares results from the at least one sensor 21 and the at least one sensor 25, thereby determining if air inside the vehicle 13 or air outside the vehicle 13 has less contaminants.

The at least one pressure sensor 27 on the interior 17 of the vehicle 13 and the at least one pressure sensor 29 on exterior 19 of the vehicle 13 read the air pressure on both the interior 17 and exterior 19 of the vehicle 13, respectively. Results from those at least one pressure sensors 27 and 29 are sent to the controller 14. The controller 14 compares results from the at least one pressure sensor 27 and the at least one pressure sensor 29 thereby determining a pressure differential between the interior 17 of the vehicle 13 and the exterior 19 of the vehicle 13. This pressure differential can influence air flow through the portal 15 when the portal 15 is opened.

If air on the exterior 19 of the vehicle 13 contains less contaminants than air on the interior 17 of the vehicle 13, the HVAC system 10 can regulate air flow between the exterior 19 of the vehicle and the interior 17 of the vehicle 13. To provide air a reduced amount of contaminants, the HVAC system 10 can operate on a substantially continuous basis to sterilize the air of the vehicle 13. The controller 14, using results from the at least one pressure sensor 27, the at least one pressure sensor 29, the at least one sensor 25 and the at least one sensor 21, regulates air pressure in the vehicle 13 to create a pressure differential favoring movement of air with reduced amount of contaminants into or out of the vehicle 13.

For instance, to prepare for an onboarding passenger to enter the vehicle 13 through the portal 15, the controller 14 uses the above-discussed results to determine if air on the interior 17 of the vehicle 13 or air on the exterior 19 of the vehicle 13 has lower contaminants. If air on the interior 17 of the vehicle 13 has lower contaminants than air on the exterior 19 of the vehicle 13, the HVAC system 10 adjusts air pressure on the interior 17 of the vehicle 13 such that when the portal 15 opens, air from the interior 17 of the vehicle 13 will move towards the exterior 19 of the vehicle 13. If air on the exterior 19 of the vehicle 13 has lower contaminants than air on the interior 17 of the vehicle, the HVAC system 10 will create reduced air pressure on the interior 17 of the vehicle 13 with respect to air pressure on the exterior 19 of the vehicle 13, thereby drawing air from the exterior 19 of the vehicle 13 to the interior 17 of the vehicle 13 through the portal 15. In either case, the air containing a higher level of contaminants moves away from the interior 17 and an occupant of the vehicle 13.

## Claims

1. A system for protecting an occupant of a vehicle (13), the system comprising:
the vehicle (13) having an interior (17) and an exterior (19);
a portal (15) disposed on the vehicle (13) through which air passes between the interior (17) of the vehicle (13) and the exterior (19) of the vehicle (13);
an HVAC system (10) disposed on the vehicle (13) for circulating air on the interior (17) of the vehicle (13);
a second sensor (25) disposed on the exterior (19) of the vehicle (19) for monitoring quality of air adjacent the second sensor (25);
a first pressure sensor (27) disposed on the interior (17) of the vehicle (13) for monitoring pressure of air adjacent the first pressure sensor (27);
a second pressure sensor (29) disposed on the exterior (19) of the vehicle (13) for monitoring pressure of air adjacent the second pressure sensor (29); and
a controller (14) connected with the first sensor (21), the second sensor (25), the first pressure sensor (27) and the second pressure sensor (29)
**characterized in**
a source (12) of electromagnetic radiation disposed in the HVAC system (10) for reducing contaminant in air;
a first sensor (21) disposed on the interior (17) of the vehicle (13) for monitoring quality of air adjacent the first sensor (21);
wherein the controller (14), using results from the at least one pressure sensor (27), the at least one pressure sensor (29), the at least one sensor (25) and the at least one sensor (21), regulates air pressure in the vehicle (13) to create a pressure differential favoring movement of air with reduced amount of contaminants into or out of the vehicle (13).

2. The system as defined in claim 1, wherein the at least one source (12) of electromagnetic radiation comprises a light emitting diode.

3. The system as defined in claim 1 or 2, wherein the electromagnetic radiation has a wavelength within a range of 200 nanometers to 280 nanometers.

4. The system as defined in any one of claims 1 to 3, wherein the electromagnetic radiation is emitted at specific times to manage vehicle (13) power usage.

## Patentansprüche

1. Ein System zum Schutz eines Insassen eines Fahrzeugs (13), wobei das System umfasst:
das Fahrzeug (13) mit einem Innenraum (17) und einem Außenbereich (19);
ein Portal (15), das an dem Fahrzeug (13) angeordnet ist und durch das Luft zwischen dem Innenraum (17) des Fahrzeugs (13) und dem Außenbereich (19) des Fahrzeugs (13) strömt;
ein HVAC-System (10), das an dem Fahrzeug (13) angeordnet ist, um Luft im Innenraum (17) des Fahrzeugs (13) zu zirkulieren;
einen zweiten Sensor (25), der am Außenbereich (19) des Fahrzeugs (19) angeordnet ist, um die Luftqualität in der Nähe des zweiten Sensors (25) zu überwachen;
einen ersten Drucksensor (27), der im Innenraum (17) des Fahrzeugs (13) angeordnet ist, um den Luftdruck in der Nähe des ersten Drucksensors (27) zu überwachen;
einen zweiten Drucksensor (29), der am Außenbereich (19) des Fahrzeugs (13) angeordnet ist, um den Luftdruck in der Nähe des zweiten Drucksensors (29) zu überwachen; und
eine Steuereinheit (14), die mit dem ersten Sensor (21), dem zweiten Sensor (25), dem ersten Drucksensor (27) und dem zweiten Drucksensor (29) verbunden ist,
**gekennzeichnet durch**
eine Quelle (12) elektromagnetischer Strahlung, die in dem HVAC-System (10) angeordnet ist, um Verunreinigungen in der Luft zu reduzieren;
einen ersten Sensor (21), der im Innenraum (17) des Fahrzeugs (13) angeordnet ist, um die Qualität der Luft in der Nähe des ersten Sensors (21) zu überwachen;
wobei die Steuereinheit (14) unter Verwendung der Ergebnisse des mindestens einen Drucksensors (27), des mindestens einen Drucksensors (29), des mindestens einen Sensors (25) und des mindestens einen Sensors (21) den Luftdruck im Fahrzeug (13) reguliert, um einen Druckunterschied zu erzeugen, der die Bewegung von Luft mit reduzierter Schadstoffmenge in das oder aus dem Fahrzeug (13) begünstigt.

2. Das System nach Anspruch 1, wobei die mindestens eine Quelle (12) elektromagnetischer Strahlung eine Leuchtdiode umfasst.

3. Das System nach Anspruch 1 oder 2, wobei die elektromagnetische Strahlung eine Wellenlänge innerhalb eines Bereiches von 200 Nanometern bis 280 Nanometern aufweist.

4. Das System nach einem der Ansprüche 1 bis 3, wobei die elektromagnetische Strahlung zu bestimmten Zeiten ausgesendet wird, um den Energieverbrauch des Fahrzeugs (13) zu steuern.

## Revendications

1. Un système destiné à protéger un occupant d'un véhicule (13), le système comprenant :
le véhicule (13) comportant un intérieur (17) et un extérieur (19) ;
un portail (15) disposé sur le véhicule (13) à travers lequel l'air passe entre l'intérieur (17) du véhicule (13) et l'extérieur (19) du véhicule (13) ;
un système CVC (10) disposé sur le véhicule (13) pour faire circuler l'air à l'intérieur (17) du véhicule (13) ;
un deuxième capteur (25) disposé à l'extérieur (19) du véhicule (19) pour surveiller la qualité de l'air à proximité du deuxième capteur (25) ;
un premier capteur de pression (27) disposé à l'intérieur (17) du véhicule (13) pour surveiller la pression de l'air à proximité du premier capteur de pression (27) ;
un deuxième capteur de pression (29) disposé à l'extérieur (19) du véhicule (13) pour surveiller la pression de l'air à proximité du deuxième capteur de pression (29) ; et
un contrôleur (14) connecté au premier capteur (21), au deuxième capteur (25), au premier capteur de pression (27) et au deuxième capteur de pression (29) **caractérisé par**
une source (12) de rayonnement électromagnétique disposée dans le système CVC (10) pour réduire les contaminants dans l'air ;
un premier capteur (21) disposé à l'intérieur (17) du véhicule (13) pour surveiller la qualité de l'air à proximité du premier capteur (21) ;
dans lequel le contrôleur (14), à l'aide des résultats provenant d'au moins un capteur de pression (27), d'au moins un capteur de pression (29), d'au moins un capteur (25) et d'au moins un capteur (21), régule la pression d'air dans le véhicule (13) afin de créer une différence de pression favorisant le mouvement de l'air avec une quantité réduite de contaminants vers l'intérieur ou l'extérieur du véhicule (13).

2. Le système selon la revendication 1, dans lequel la au moins une source (12) de rayonnement électromagnétique comprend une diode électroluminescente.

3. Le système selon la revendication 1 ou 2, dans lequel le rayonnement électromagnétique a une longueur d'onde comprise entre 200 nanomètres et 280 nanomètres.

4. Le système selon l'une des revendications 1 à 3, dans lequel le rayonnement électromagnétique est émis à des moments spécifiques afin de gérer la consommation d'énergie du véhicule (13).
